## Europäisches Patentamt

(19) **European Patent Office**

**Office européen des brevets**

(11) Publication number: **0 032 210**
**B1**

(12) **EUROPEAN PATENT SPECIFICATION**

(45) Date of publication of patent specification: **14.11.84**

(21) Application number: **80107844.5**

(22) Date of filing: **12.12.80**

(51) Int. Cl.³: **C 07 B 9/00, B 01 J 12/00, B 01 J 19/24** // C07C19/08, C07C49/167

(54) **Fluorination of organic compounds with fluorine in porous metal tube reactor with perfluorinated diluent.**

(30) Priority: **26.12.79 US 107122**
**26.12.79 US 107123**

(43) Date of publication of application:
**22.07.81 Bulletin 81/29**

(45) Publication of the grant of the patent:
**14.11.84 Bulletin 84/46**

(84) Designated Contracting States:
**DE FR GB IT**

(56) References cited:
CA-A- 990 738
DE-C- 651 049

CHEMICAL ABSTRACTS, vol.80, 1974, page 371, abstract 95458u Columbus, Ohio. USA
Ullmanns Encyclopädie der technischen Chemie Bd. 11 (4. Aufl., Verlag Chemie, Weinheim) S. 589
W.A. Sheppard, Organic Fluorine Chemistry (W.A. Benjamin Inc, 1969) p. 52-53

(73) Proprietor: **ALLIED CORPORATION**
**Columbia Road and Park Avenue P.O. Box 2245R (Law Dept.)**
**Morristown New Jersey 07960 (US)**

(72) Inventor: **Robinson, Martin Alvin**
**167 Wood Acres Drive**
**East Amherst New York 14051 (US)**
Inventor: **Nychka, Henry Robert**
**180 Hillcrest Road**
**East Aurora New York 14052 (US)**
Inventor: **Hino, John Bernhard**
**92 Southgate Road**
**Cheektowaga New York 14215 (US)**
Inventor: **Eibeck, Richard Elmer**
**23 Pine Terrace**
**Orchard Park New York 14127 (US)**
Inventor: **Brauman, John I**
**849 Tolman Drive**
**Stanford California 94305 (US)**

(74) Representative: **Collier, Jeremy Austin Grey et al**
**J.A.Kemp & Co. 14, South Square Gray's Inn London WC1R 5EU (GB)**

Courier Press, Leamington Spa, England.

**0 032 210**

## Description

Background of the invention

This invention relates to the fluorination of organic compounds with elemental fluorine and particularly to the fluorination of organic compounds in a porous tube reactor wherein fluorine is fed onto one side of a porous member and the organic compound is fed onto the other side of the porous member, fluorine migrates through the porous member and fluorinated products are recovered from the other side of the porous member.

The direct fluorination of organic compounds using elemental fluorine as a reactant, especially where the organic compound contains a carbon backbone of two or more atoms, has been considered impractical. Thus standard texts indicate the reaction $R—H+X_2 \rightarrow R—X+HX$ to be satisfactory only when X is Cl or Br and not when X is F. Especially where R has more than one carbon, a substantial proportion of products having a different number of carbons than the reactants has been produced. Thus, for example, in the literature reports in volumes 61, p. 3552—3554, 62, p. 267—269 and 63, p. 788—791 and 2792—2795 of the Journal of the American Chemical Society by L. A. Bigelow, yields with methane, ethane and acetone of $CF_4$, $C_2F_2$ and $CF_3COCF_3$ were, respectively, 30—50%, 30—40% and 10%. Intermediates and by-products for methane included $CH_{1-3}F_{3-1}$, $C_2F_6$ and $C_3F_8$. Intermediates and byproducts for ethane included $C_2H_{1-5}F_{5-1}$ $CH_{1-3}F_{3-1}$, $C_2F_6$ and $CF_4$ (the latter resulting from cleavage of a carbon carbon bond). Intermediate and byproducts for acetone included $CF_4$, $COF_2$, $CF_3COF$, $(COF)_2$ and $CH_3COCH_2F$.

An invention of Bruce E. Kurtz, described in Canadian Patent 990,738 issued June 6, 1976 and also described in a somewhat different form in U.S. Patent 4,187,253, issued February 5, 1980, includes the chlorination of one and two carbon hydrocarbons and their partially chlorinated derivatives in a porous tube reactor. In these references the porous tube is an elongated member, preferably of fritted Pyrex glass (Pyrex being a registered trademark), but also permissibly of Alundum (a registered trademark for a fixed alumina refractory material), a ceramic or a sintered metal, being in each case resistant to chlorine, anhydrous HCl, and the feed and product organic and chlorinated organic compounds.

Two Japanese patents disclose the fluorination of organic compounds in a solvent with fluorine, using sulfur hexafluoride as a diluent for the fluorine. In Kokai 49000,201 (January 5, 1974) (Chemical Abstracts 80:95458n) non-polymeric organic compounds such as $C_6H_6$, toluene and uracil were used as reactants with the corresponding monofluorocompounds produced. In 49046281 (December 9, 1974) (Chemical Abstracts 82:173168) carbon and fluorine in the presence of $SF_6$ produced "$(CF)_m$" polymer and $CF_4$ as a minor by-product.

Various processes have been proposed for electrochemical chlorination and fluorination of organic compounds. Organic compounds dissolved in or bubbled into anhydrous HF or molten fluoride salt-HF combinations (e.g. KF-2HF) are subjected to an electrolytic voltage, forming elemental fluorine at the anode which fluorinates the product. U.S. Patents 2,841,544 and 3,298,940 describe such a process with a porous or foraminiferous anode, preferably made of carbon. In various Phillips Petroleum Company patents, the organic material is fed into the porous carbon anode and elemental fluorine passed from the electrolyte at the anode surface (where it is formed) into the porous carbon anode. Helium is sometimes used as a diluent.

The present invention relates to the production of perfluoropropane or octafluoropropane which is known to be useful in plasma etching of electronic components and is a dielectric gas.

The production of perfluoropropane has been attempted from a variety of starting materials, but no process has been developed which produces the material in high yields without a multitude of steps. Exemplary starting materials are propane, propene, hexafluoropropene and partially halogenated propanes and propenes.

U.S. Patent 4,158,023 to *von Halasz* describes the production of perfluoropropane from hexafluoropropene in two steps. This reference indicates that the direct conversion with elemental fluorine results in poor yields. Since hexafluoropropene is itself fairly hard to produce in good yields, the overall process becomes involved with a multitude of steps and expense.

U.S. Patent 3,840,445 to *Paul et al.* describes electrochemical fluorination of propane to perfluoropropane. The reference indicates the difficulty in separating propane from the product by distillation, and, accordingly, employs a two-step process of electrochemical fluorination. Although the product need be separated only from partially fluorinated propane when the two steps are used, the process thereby becomes more involved, and the potential for overall yield losses increases.

The production of perfluoropropane from propane by elemental fluorination in a jet reactor has been described in an article by A. F. Maxwell et al. in *Journal of the American Chemical Society*, Vol. 82, pp. 5827—30 (November 20, 1960). The tables in this article report production of perfluoropropane which suggest a yield, in the base case, of about 50 percent based on propane fed.

DE—C—651049 describes the fluorination of organic compounds in the liquid phase using a tube of inert material, such as iron, nickel or copper, which is provided with small openings and is arranged in a rotating drum containing the compound to be fluorinated, either as such or dissolved in a solvent, in an amount insufficient for direct contact with the tube. Fluorine is introduced through the

2

**0 032 210**

tube and exits through the openings and contacts the compound in areas containing inert solid materials.

The present invention provides a process for perfluorinating acyclic organic compounds which comprises:

a) passing fluorine as a first gaseous reactant into an elongated inner zone surrounded by a porous metal member having a pore size of between 0.5 and 50 $\mu$m and being substantially resistant to fluorine corrosion and

b) introducing as a second gaseous reactant outside the porous metal member at least one acyclic hydrocarbon of 2 to 8 carbons or ketone of 3 to 8 carbons, or partially halogenated derivative of either of these, each such compound having at least one fluorinatable carbon selected from carbon atoms covalently bonded to H, Cl or Br and carbons olefinically or acetylenically bonded to other carbons, the pressure in said elongated inner zone being greater than the pressure outside the porous metal member, and at least one of said first and second gaseous reactants being mixed with a perfluorinated gaseous diluent selected from sulfur hexafluoride, tetrafluoromethane, hexafluoroethane and octafluoropropane.

It has been discovered that the combination of the porous metal tube and the use of the perfluorinated diluent such as sulfur hexafluoride enables such fluorinations to occur with a maximum yield of perfluorinated products or like carbon number and a minimum degree of cleavage products with lesser carbon numbers.

A preferred class of acrylic organic compounds are those with three carbons, such as propane, propene or hexafluoropropene, with the product octafluoropropane being recovered from the effluent outside the porous member.

The present invention employs a porous metal tube for the elemental fluorination of organic compounds. When using such a tube for fluorination, unlike the fluorinations described in Canadian Patent 990,738 and U.S. Patent 4,187,253, it is not sufficient to employ common inert diluents such as nitrogen and achieve good yields with low losses to cleavage reactions or carbon formation. Instead it has been found that the perfluorinated diluent such as sulfur hexafluoride is required.

The organic compound used as a second gaseous reactant must contain at least one carbon atom which is "fluorinatable", by which is meant a carbon atom having an H, Cl or Br attached that can be replaced by F or an olefinic or acetylenic bond to another carbon that can be added across by fluorine. Compounds having at least one C-H bond are preferred over compounds having only fluorinatable carbons other than carbons bonded to hydrogen. While carbons bonded to "hetero" atoms such as Br, Cl or O are eventually fluorinated, the process of the invention can also be made selective by using limited amounts of fluorine and/or mild conditions so as to preserve hetero atoms and replace only hydrogens and unsaturations by fluorine. By "perfluorination" we therefore mean that at least all C-H bonds and carbon-carbon-unsaturations in the second gaseous reactant are replaced by C-F bonds.

One group of second reactants are the aliphatic hydrocarbons of two to eight carbons, including ethane, propane and butane and olefinic hydrocarbons such as ethene, propene and butene. These materials can be converted to the corresponding perfluoroalkanes of the formula $C_nF_{2n+2}$. Propane and propene are two preferred reactants in the group.

Another group of second reactants are partially halogenated derivatives of the aliphatic hydrocarbons having some or all of the hydrogens or unsaturations replaced by Cl, Br or F, but not all by F. Only hydrogens and unsaturations of such reactants are replaced by fluorine under mild conditions, especially temperature, and when the amount of fluorine is limited to that required for the desired fluorination. Thus, $C_2H_5Cl$ can be converted to $C_2ClF_5$ with low temperatures and only five moles of fluorine per $C_2H_5Cl$; or it can be converted to $C_2F_6$ with six or more moles of fluorine per $C_2H_5Cl$.

Another group of second reactants are the ketones of 3—8 carbons such as acetone, methyl ethyl ketone and higher members of the series, together with partially halogenated ketones having some or all of the hydrogens replaced by Cl, Br or F, but not all by F. In general hydrogens will first be replaced by fluorine, then Br, then Cl and only then the carbonyl oxygen. Preferred is acetone and partially halogenated acetone.

Either the first or second gaseous reactant can be mixed with the perfluorinated diluent, such as sulfur hexafluoride. Preferably, both reactants are mixed with sulfur hexafluoride. The mole ratio of fluorine to sulfur hexafluoride mixed therewith is preferably between 2:1 and 1:12, more preferably between 1:5 and 1:10. The mole ratio of second gaseous reactant to sulfur hexafluoride mixed therewith is preferably between 4:1 and 1:5, more preferably between 1:1.5 and 1:3.

The ratio of fluorine to three carbon compound depends, in part, upon the particular three carbon compound employed. Thus, in general, one can compute the stoichiometric amount of fluorine required to convert the entire second reactant to a perfluoropropane as a first estimate of the desired amount of fluorine to be introduced.

For three carbon compounds having a hydrogen, one mole of fluorine is required for each mole of hydrogen bonded to carbon such that half of the fluorine molecule can replace the hydrogen to form a C-F bond and the other half of the fluorine molecule can form HF byproduct. For each unsaturation, one

3

mole of fluorine is required to add across a double bond and to form two C-F bonds. For each other halogen present such as Cl or Br, or any other hetero atom present, one molecule of fluorine is generally required to both replace the hetero atom and form a by-product between fluorine and the hetero atom. In general, however, hetero atoms are preferably not present such that the three carbon compound has no elements other than carbon, hydrogen and fluorine.

While any amount of fluorine may be used, it is preferred to use between 50 and 150 percent of the stoichiometric amount. Thus, in the case of propane, 8 moles of fluorine per propane constitutes the stoichiometric amount, and a generally suitable range of fluorine is between 4 and 12 moles of fluorine per propane. Preferably, between 5 and 10 moles of fluorine are intended into the inner zone per mole of propane introduced outside the elongated porous member. More preferably between 8 and 9.5 moles of fluorine are introduced per mole of fluorine.

In the case of propane, the stoichiometric amount is 7 moles of fluorine per propene. A generally suitable overall range is between 3.5 and 10.5 moles of fluorine per mole of propene. A preferred range of fluorine to propene ratios is between 4:1 and 8:1, more preferably between 7:1 and 8:1.

In the case of hexafluoropropene, the stoichiometric mole is 1:1 while a range of 0.5:1 to 1.5:1 is suitable, as well as amounts higher, and especially lower in fluorine than that range, the preferred ratios are between 0.7:1 and 1.1:1, especially about 1:1.

The porous tube used in the present invention can be any metal inert to corrosion by fluorine under the relatively mild reaction conditions employed. Preferably the pore size is between 0.5 and 50 micrometers, taken as an average of all pores or as a limitation on substantially each and every pore.

Where propane is the reactant, octafluoropropane can be recovered from the effluent by condensing the effluent under sufficiently high pressure and low temperature to condense octafluoropropane, separating the condensate from the uncondensed portion of the effluent, separating an organic phase of the condensate from a hydrogen fluoride phase of the condensate and recovering octafluoropropane from the organic phase of the condensate. The preferred method of recovering octafluoropropane from the organic phase of the condensate is by fractional distillation since, once phase separation has occurred, no compounds boiling close to octafluoropropane will remain in the condensate. It should be appreciated that, if propane itself were present in the effluent, it would also be found in the organic phase of the condensate and would, according to U.S. Patent 3,840,445 complicate purification. In the examples described below having a stoichiometric excess of fluorine, no propane was detected in the effluent and no difficulty should be encountered in separating perfluoropropane from other materials by fractional distillation.

Example 1

Fluorination of ethane

Fluorine gas (0.1 mol/h) admixed with sulfur hexafluoride (0.50 mol/h) was introduced at 101.4 kPa pressure into the bottom of a 6 inch (15.2 cm) long, 1.5 inch diameter Inconel porous tube of wall thickness 0.065 inch (0.165 cm) thickness and 10 micrometer pore size (Inconel being a registered trademark for a nickel-ironchromium alloy). The tube was sealed at the top. Ethane (0.40 mol/h) was introduced at a pressure of 101.4 kPa into a 1/2 inch (1.27 cm) by six inch (15.3 cm) annular zone outside the porous tube and inside a concentric impervious stainless steel tube 2 inches (5.08 cm) in diameter. Gaseous effluent was removed from the top of the annular zone. Thus total feeds were 0.10 mol/h $F_2$, 0.40 mol/h of $C_2H_6$ and 0.50 mol/h of $SF_6$. The temperature was monitored by a thermowell along the outside of the porous tube and showed a maximum of 76°C at a calculated contact time of 20 seconds. On-line gas chromatographic analysis of the effluent, after passing through a sodium fluoride bed to absorb the HF, showed about .013 mol/h $C_2F_6$ (80% yield based on $F_2$ feed), about .005 mol/h $CF_4$ (20% yield based on $F_2$ feed) and no partially fluorinated ethanes such as $C_2HF_5$ or $C_2H_5F$.

A similar experiment using nitrogen as the diluent produced only $CF_4$, the cleavage product, and no measurable $C_2F_6$. In this experiment the maximum temperature sensed by the thermowell was 75°C. Even when the reaction temperature was lowered to −50°C by a cooling bath only $CF_4$ was observed in the effluent. Those two runs are described in more detail as Comparative Examples 15 and 16, below.

Examples 2—4

Fluorination of propane and propene

Example 1 was repeated using the same tube, but now feeding 0.25 or 0.50 mol/h of $F_2$ admixed with 2.0 mol/h of $SF_6$ into the bottom of the porous tube and 0.25 mol/h of propane or propene admixed with 0.50 mol/h of $SF_6$ into the annular zone, all as shown in Table 1. On-line gas chromatographic analysis of the vaporized effluent gave the area percentages shown in Table 1.

TABLE 1

Fluorination of acetone in pipe reactor

| Example | 2 | 3 | 4 |
|---|---|---|---|
| Feeds C-3 Organic (0.25 mol/h) | propene | propane | propane |
| $SF_6$ (mol/h) annular zone | 2.0 | 2.0 | 2.0 |
| $F_2$ (mol/h) | 0.25 | 0.25 | 0.50 |
| $SF_6$ (mol/h) inside tube | 0.50 | 0.50 | 0.50 |
| $CF_4$ (area %) | 3 | 0 | 3 |
| $C_3F_8$ (area %) | 18 | 18 | 58 |
| $C_3H_{1-7}F_{7-1}$*(area %) | 79 | 82 | 39 |
| $C_3F_8$ yield (based on $F_2$) | 18 | 17 | 54 |

\* together with similar partially fluorinated hexanes

Example 5
Fluorination of acetone

Using the apparatus of Example 1, 0.25 mol/h of fluorine admixed with 2.0 mol/h of sulfur hexafluoride was admitted into the porous tube and 0.30 mol/h of acetone and 0.60 mol/h of sulfur hexafluoride was admitted to the annular zone. This reaction was continued for ten hours with the effluent passing through an NaF scrubber to remove HF by absorption and then trapped in condenser traps at $-80°C$ and $-196°C$. The temperature profile measured by the thermowell along the surface of the porous tube under steady state conditions was $117°C$ at the base, $139°C$ two inches (5 cm) from the base, $141°C$ four inches (10 cm) above the base and $125°C$ at the top of the tube six inches (15 cm) above the base. The thermowell measured $73°C$ two inches (5 cm) above the upper end of the porous tube.

The trapped materials were combined into one cylinder which contained 100 g of water. The purpose of the water was to complex the fluoroacetones as their hydrates which could then be isolated by distillation. After venting off the more volatile $SF_6$, the recovered aqueous organic mixture which was composed of unreacted acetone, $H_2O$ and fluoroacetone hydrates was distilled. The comparative results of distillation and gas chromatographic analysis of the aqueous organic mixture (expressed as the anhydrous products) are shown in Table 2.

TABLE 2

Fluorination of acetone

|  | Distillation | Gas chromatography |
|---|---|---|
| Hexafluoroacetone Yield % | 36.8 | 50.2 |
| Pentafluoroacetone | 3.6 | 2.1 |
| Tetrafluoroacetone (sym) | 0.4 | 7.4 |
| Difluoroacetone (sym) | 0.1 | — |
| Monofluoroacetone | 4.6 | 6.7 |
| Total yield ($F_2$ basis) | 45.5 | 66.4 |
| HF Yield, % | 86.3 |  |

The yield obtained for distillation is appreciably lower due to decomposition during the distillation.

Examples 6—12
Fluorination of acetone with different porous tubes

Example 5 was repeated for shorter runs with the effluent analyzed by on-line gas chromatography. In Examples 6—8 a porous stainless steel (SS) tube with 0.5 micrometer pore size was used. In Examples 9—11 a porous nickel (N) tube with 5 micrometer pore size was used. Both tubes have substantially the same external dimensions and thickness as the Inconel (I) tube used in Examples 1—5 and also in Example 12. The feed rates into the inner zone (IZ) and annular zone (AZ), contact times, maximum temperature measured by the thermowell and product analyses (by area % normalized to exclude unreacted acetone and sulfur hexafluoride) are all shown in Table 3. Also shown is the HF in the effluent as a percentage of the theoretical value obtained by assuming that each fluorine molecule fed was involved in the stoichiometry $R-H+F_2 \rightarrow R-F+HF$.

TABLE 3

| Example | 6 | 7 | 8 |
|---|---|---|---|
| Tube | SS | SS | SS |
| IZ Flow ($F_2/SF_6$) | 0.09/0.5 | 0.3/1.0 | 0.15/0 |
| AZ Flow (Acetone/$SF_6$) | 0.32/0.61 | 0.32/0.61 | 0.29/1.61 |
| Contact Time (S) | 12.6 | 7.3 | 8.5 |
| Max. Temp. (°C) | 100 | 185 | 141 |
| GC Analysis (%) $CF_3COCF_3$ | 62 | 9 | 9 |
| Other Fluoro-Acetones | 14 | 67 | 10 |
| $CF_4$ | 18 | 18 | 61 |
| $COF_2$* | 6 | 6 | 20 |
| HF (% of Theory) | 90 | — | 79 |

| Example | 9 | 10 | 11 | 12 |
|---|---|---|---|---|
| Tube | N | N | N | I |
| IZ Flow ($F_2/SF_6$) | 0.13/1.0 | 0.24/1.0 | 0.24/1.0 | 0.25/2.0 |
| AZ Flow (Acetone/$SF_6$) | 0.30/0.61 | 0.30/0.61 | 0.30/0.30 | 0.30/0.61 |
| Contact Time (S) | 5.4 | 7.5 | 8.6 | 5.4 |
| Max. Temp. (°C) | 85 | 113 | 122 | 136 |
| GC Analysis (%) $CF_3COCF_3$ | 65 | 49 | 20 | 62 |
| Other Fluoro-Acetones | 19 | 11 | 5 | 20 |
| $CF_4$ | 12 | 30 | 51 | 13 |
| $COF_2$* | 4 | 10 | 18 | 5 |
| HF (% of Theory) | 76 | — | 100 | 83 |

* The $COF_2$ value is estimated because of interference between the $COF_2$ peak and the $SF_6$ peak in the analysis.

Example 8 represents a less preferred mode of the process in which acetone, but not fluorine, was admixed with sulfur hexafluoride before feeding into the reactor. This example produced the highest proportion of cleavage products ($CF_4$ and $COF_2$). Examples 6, 9 and 12 represent the more preferred modes of the invention where the fluorine to sulfur hexafluoride molar ratio fed to the inner zone is between about 1:5 and about 1:10 (1:5.5, 1:7.7 and 1/8 respectively). Examples 7, 10 and 11 have slightly less preferred molar ratios of fluorine to sulfur hexafluoride (1:3.3, 1:4.2 and 1:4.2 respectively) which is still within the broader range of between about 2:1 and about 1:12.

Comparative Examples 13 and 14
Fluorination of methane
        Using an Inconel porous tube with 10 micrometer pore size, and otherwise the same apparatus as in Example 1, methane was fluorinated using nitrogen as the diluent. $CF_4$, but no partially fluorinated methanes were observed in the effluent by on-line gas chromatography. In Comparative Example 13 the total feeds were 0.5 mol/h of fluorine, 0.45 mol/h of methane and 0.5 mol/h of nitrogen. The respective feeds in Comparative Example 14 were 0.1, 0.4 and 0.5 mol/h. The maximum temperatures observed by the thermowell were 52°C and 69°C, respectively. Calculated yields were 88% and 84% tetrafluoroethane, respectively. This comparative example indicates that if one were to apply the teachings of Canadian Patent 990,738 directly to fluorination of methane, reactivity would be observed with a conventional nitrogen diluent.

Comparative Examples 15 and 16
Fluorination of ethane
        Several attempts were made to fluorinate ethane with fluorine using nitrogen as the diluent. Two such examples are described herein. Using the Inconel porous tube described above, 0.5 mol/h of ethane was fed into the outer zone of the reactor and 0.1 mol/h of $F_2$ diluted with 0.5 mol/h of nitrogen was fed into the annular zone. Maximum temperature observed was 74°C. Gas chromatographic analysis of effluent showed no $C_{2F6}$ but essentially all $CF_4$. The yield of the latter agreed with $F_2$ introduced. The experiment was repeated except that the reaction was immersed in a cooling bath to give an internal temperature of about −50°. The results were the same as the preceding.

Comparative Example 17
        An attempt was made to fluorinate acetone with fluorine, diluting each with sulfur hexafluoride, in a pipe reactor. A cylindrical stainless steel pipe one inch (2.5 cm) inside diameter by 12 inch (30 cm) length was packed with 27.5 g copper mesh. Streams of 0.07 mol/h of fluorine diluted with 0.5 mol/h of sulfur hexafluoride and 0.25 mol/h of acetone diluted with 0.61 mol/h of sulfur hexafluoride were fed into the bottom end and the effluent from the top end was analyzed by on-line gas chromatography. The maximum temperature sensed by a thermowell along the center of the reactor was 100°C. Partially fluorinated acetones, but no $CF_3COCF_3$ and no $CF_4$ were detected in the effluent.

Comparative Example 18
        When Comparative Example 17 was repeated at higher flow rates of fluorine and consequently higher temperatures, increasing amounts of both $CF_3COCF_3$ and $CF_4$ were observed. Several runs are plotted in Table 4. This example shows that, while some benefits can be achieved by using $SF_6$ as a diluent in a pipe tube reactor, higher amounts of cleavage products are obtained compared to $CF_3COCF_3$ than in the porous tube reactor process employed in Examples 5—12.

TABLE 4
Fluorination of acetone in pipe reactor

| Run | 17 | 18A | 18B |
|---|---|---|---|
| $F_2/SF_6$ flow (mol/h) | 0.07/0.50 | 0.13/0.50 | 0.25/0.50 |
| Acetone/$SF_6$ flow (mol/h) | 0.25/0.61 | Same | Same |
| Contact Time (s) | 17.2 | 16.4 | 15.3 |
| Max. Temp. °C | 100 | 170 | 250 |
| GC Quantitative Analysis $CF_3COCF_3$ | nil | low | nil |
| Other Fluoro-Acetones | high | medium | low |
| $CF_4$ and $COF_2$ | low | medium | high |

## 0 032 210

### Claims

1. A process for perfluorinating acyclic organic compounds in the gas phase which comprises:

a) passing fluorine as a first gaseous reactant into an elongated inner zone surrounded by a porous metal member having a pore size of between 0.5 and 50 $\mu$m and being substantially resistant to fluorine corrosion; and

b) introducing as a second gaseous reactant outside the porous metal member at least one acyclic hydrocarbon of 2 to 8 carbons or ketone of 3 to 8 carbons, or partially halogenated derivative of either of these, each such compound having at least one fluorinatable carbon selected from carbon atoms covalently bonded to H, Cl or Br and carbons olefinically or acetylenically bonded to other carbons, the pressure in said elongated inner zone being greater than the pressure outside the porous metal member, and at least one of said first and second gaseous reactants being mixed with a perfluorinated gaseous diluent selected from sulfur hexafluoride, tetrafluoromethane, hexafluoroethane and octafluoropropane.

2. A process according to claim 1 wherein both the first and second gaseous reactants are mixed with sulfur hexafluoride as gaseous diluent.

3. A process according to claim 2 wherein the mole ratio of fluorine in the first gaseous reactant to sulfur hexafluoride mixed therewith is between 2:1 and 1:12 and the mole ratio of second gaseous reactant to sulfur hexafluoride mixed therewith is between 4:1 and 1:5.

4. A process according to claim 1, 2 or 3 wherein the second gaseous reactant is an acyclic hydrocarbon having at most one olefinic unsaturation.

5. A process according to any one of the preceding claims wherein the second gaseous reactant has three carbons.

6. A process according to claim 5 wherein the second gaseous reactant is propane or propene.

7. A process according to any one of the preceding claims wherein between 50 and 150 percent of the stoichiometric amount of fluorine is used.

### Patentansprüche

1. Verfahren zur Perfluorierung acyclischer organischer Verbindungen in der Gasphase, indem man

a) Fluor als einen ersten gasförmigen Reacktionspartner in eine längliche innere Zone einführt, die von einem porösen Metallteil umgeben ist, das eine Porengröße zwischen 0,5 und 50 $\mu$m besitzt und gegen Fluorokorrosion im wesentlichen beständig ist, und

b) als einen zweiten gasförmigen Reaktionspartner außerhalb des porösen Metallteils wenigstens einen acyclischen Kohlenwasserstoff mit 2 bis 8 Kohlenstoffatomen oder ein Keton mit 3 bis 8 Kohlenstoffatomen oder eine teilhalogeniertes Derivat einer dieser Verbindungen einführt, wobei diese Verbindung wenigstens ein fluorierbares Kohlenstoffatom aus der Gruppe der kovalent am H, Cl order Br gebundenen Kohlenstoffatome und der olefinisch oder acetylenisch an andere Kohlenstoffatome gebundenen Kohlenstoffatome ausgewählt ist, der Druck in der länglichen inneren Zone größer als der Druck außerhalb des porösen Metallteils ist und wenigstens einer der ersten und zweiten gasförmigen Reaktionspartner mit einem perfluorierten gasförmigen Verdünnungsmittel vermischt ist, das aus der Gruppe Schwefelhexafluorid, Tetrafluormethan, Hexafluoräthan und Octafluorpropan ausgewählt ist.

2. Verfahren nach Anspruch 1, bei dem der erste und der zweite gasförmige Reaktionspartner mit Schwefelhexafluorid als gasförmiges Verdünnungsmittel vermischt ist.

3. Verfahren nach Anspruch 2, bei dem das Molverhältnis von Fluor in dem ersten gasförmigen Reaktionspartner zu mit ihm vermischtem Schwefelhexafluorid zwischen 2:1 und 1:2 liegt und sas Molverhältnis von zweiten gasförmigem Reaktionspartner zu mit ihm vermischtem Schwefelhexafluorid zwischen 4:1 und 1:5 liegt.

4. Verfahren nach Anspruch 1, 2 oder 3, bei dem der zweite gasförmige Reaktionspartner ein acyclischer Kohlenwasserstoff mit höchstens einer olefinisch ungesättigten Bindung ist.

5. Verfahren nach einem der vorausgehenden Ansprüche, bei dem der zweite gasförmige Reaktionspartner 3 Kohlenstoffatome besitzt.

6. Verfahren nach Anspruch 5, bei dem der zweite gasförmige Reaktionspartner Propan oder Propen ist.

7. Verfahren nach einem der vorausgehenden Ansprüche, bei dem zwischen 50 und 150% der stöchiometrischen Menge an Fluor verwendet werden.

**Revendications**

1. Procédé pour perfluorer des composés organiques acycliques en phase gazeuse qui consiste:

a) à faire passer le fluor en tant que premier réactif gazeux dans une zone interne allongée entourée par un élément métallique poreux d'une porométrie comprise entre 0,5 et 50 $\mu$m et résistant bien à la corrosion par le fluor et

b) à introduire en tant que deuxième réactif gazeux, à l'extérieur de l'élément métallique poreux, au moins un hydrocarbure acyclique à 2 à 8 carbones ou une cétone à 3 à 8 carbones ou un dérivé partiellement halogéné de l'un des précédents chacun de ces composés ayant au moins un carbone susceptible d'être fluoré choisi dans les atomes de carbone à liaison à covalence avec H, CL ou Br et des carbones à liaison oléfinique ou acétylénique avec d'autres carbones, la pression dans ladite zone interne allongée étant supérieure à la pression extérieure régnant dans l'élément métallique poreux; et au moins un desdits premier et deuxième réactifs gazeux étant mélangés à un diluant gazeux perfluoré choisi parmi l'hexafluorure de soufre, le tétrafluorométhane, l'hexafluoroéthane et l'octafluoropropane.

2. Procédé selon la revendication 1 dans lequel le premier et le deuxième réactifs gazeux sont mélangés à de l'hexafluorure de soufre en tant que diluant gazeux.

3. Procédé selon la revendication 2 dans lequel le rapport molaire de fluor dans le premier réactif gazeux à l'hexafluorure de soufre mélangé avec lui est compris entre 2:5 et 1:12 et le rapport molaire du deuxième réactif gazeux à l'hexafluorure de soufre mélangé avec lui est compris entre 4:1 et 1:5.

4. Procédé selon la revendication 1, 2 ou 3 dans lequel le deuxième réactif gazeux est un hydrocarbure acyclique ayant au plus une insaturation oléfinique.

5. Procédé selon l'une quelconque des revendications précédentes dans lequel le deuxième réactif gazeux a trois carbones.

6. Procédé selon la revendication 5 dans lequel le deuxième réactif gazeux est le propane ou le propène.

7. Procédé selon l'une quelconque des revendications précédentes dans lequel la quantité de fluor utilisée est comprise entre 50 et 150% de la quantité stoechiométrique.